# EUROPEAN PATENT APPLICATION

(11) **EP 2 700 931 A1**
(43) Date of publication of application: **26.02.2014**
(21) Application number: 12774640.2
(22) Date of filing: 20.04.2012
(51) Int. Cl.: G01N 3/20, A61C 13/083, A61F 2/30, A61F 2/36, G01N 29/14

(54) **STRENGTH GUARANTEE TEST METHOD AND APPARATUS USED THEREIN**

(30) Priority: 22.04.2011 JP 2011096038
(71) Applicant: Tokyo Metropolitan University, Tokyo 163-8001 (JP); Kyocera Medical Corporation, Osaka-shi, Osaka 532-0003 (JP)
(72) Inventor: WAKAYAMA, Shuichi, Hachioji-shi, Tokyo 192-0397 (JP); IKEDA, Junji, Osaka-shi, Osaka 532-0003 (JP)
(74) Representative: Müller-Boré & Partner Patentanwälte PartG mbB
(86) International application number: PCT/JP2012/060735
(87) International publication number: WO 2012/144606

(57) **Abstract**

The present invention is intended for improving the reliability of a strength proof test method for ceramic parts. The present invention is directed to a strength proof test method for making total inspection of ceramic parts, including: applying a prescribed load to the ceramic parts; and at the same time, making an acoustic emission measurement, in which the ceramic parts are considered as passing when the ceramic parts are not fractured in the test and the variation of acoustic emission energy is not more than a threshold.

## Description

### TECHNICAL FIELD

The present invention relates to a proof test, and more particularly, it relates to a strength proof test method for ceramic parts particularly sensitive to defects and a device to be used therein.

### BACKGROUND ART

Ceramic materials are excellent in characteristics such as hardness, in vivo stability, and heat resistance; therefore, the field of their application involves aerospace materials and biomaterials. However, ceramic materials are more sensitive to defects as compared with metal or other materials, and their fracture probability is known to conform to the Weibull distribution.

When ceramic materials are used as, for example, biomaterials, they are widely used as, for example, artificial joint prostesis or dental implant, and since they have excellent sliding characteristics and wear resistance, they are preferably used as artificial femoral heads, acetabular liners, knee joint femoral component, and others, in particular. However, ceramic materials used as such biomaterials have a risk of in vivo fracture, and even in clinical practice, for example, the facture of femoral head or others is sometimes reported, which has become an important problem to be solved. The rate of the in vivo facture of ceramic head is said to be about 0.022% for high-purity alumina and about 0.003% for zirconia-toughened alumina.

To reduce such a risk of fracture, a proof test (also called as a proof test) is carried out in ordinary cases. In the proof test, a load assumed in the use of a product is applied to the product, and a damaged product is removed. For example, the proof test of femoral head or acetabular liners is carried out by applying a load to the femoral heads using water pressure or the like to reproduce the stress state similar to actual use, based on the CCC method in ISO7206-10.

For example, patent documents 1 and 2 disclose proof test methods for hip joint prosthesis or others. A load is applied by the compression of a polymer in patent document 1 or by a polymer, a pressure liquid, or the like in patent document 2.

### PRIOR ART DOCUMENTS

### PATENT DOCUMENTS

Patent Document 1: Japanese Patent Laid-open Publication (Kokai) No. 2009-61069
Patent Document 2: Japanese Patent Laid-open Publication (Kohyo) No. 2002-525569

### SUMMARY OF THE INVENTION

### PROBLEMS TO BE SOLVED BY THE INVENTION

However, delayed fracture often seen in ceramics has not been considered in the proof tests that have hitherto been proposed, as in patent documents 1 and 2 described above. In addition, the proof tests that have hitherto been proposed are intended for removing products fractured in the test. In other words, it has not been considered that even if products are not fractured in the proof test, damage may possibly be introduced into the products by a load in the proof test and this or other reasons rather lower the load resistant strength of the products, resulting in a risk that the products may be fractured in actual use.

The present invention has been made in the above circumstances, and the object thereof is to improve the reliability of a strength proof test method for ceramic parts.

### MEANS FOR SOLVING THE PROBLEMS

The present inventors have advanced their own research on the occurrence of acoustic emission (AE) events in the fracture process of ceramics. The AE method was disclosed in, for example, "Tsuneo KANASUGI and Shuichi WAKAYAMA, Evaluation of microscopic fracture process in compression test of ceramic femoral heads by AE method, Proceedings of the 47th Undergraduate Student Research Presentation in the Student Association of Kanto Branch, Tokyo (2008)" and "Shuichi WAKAYAMA et al., Quantitative Detection of Microcracks in Bioceramics using AE Source Characterization, Proceedings of the SEM Annual Conference, New Mexico USA (2009)." The present inventors have found a method of specifically applying the AE method to proof tests as a means of solving the problem that the introduction of damage in the previous proof tests makes their reliability insufficient, thereby completing the present invention.

The present invention, which achieved the above problem, is directed to a strength proof test method for making total inspection of ceramic parts before use, comprising: applying a prescribed load to the ceramic parts; and at the same time, making an acoustic emission measurement, in which the ceramic parts shall be considered as passing when the ceramic parts are not fractured in the test and the variation of acoustic emission energy is not more than a threshold. In the present invention, the location of acoustic emission occurrence may preferably be identified using two or more acoustic emission sensors.

In the present invention, the ceramic parts may preferably be analyzed for stress distribution state in actual use by the finite element method to determine a stress concentration region in the ceramic parts, and the ceramic parts may preferably be considered as passing when the ceramic parts are not fractured in the test and the acoustic emission energy occurring in the stress concentration region is not more than a threshold.

Examples of the ceramic parts may include in vivo implant. The in vivo implant may include artificial joint prostesis and dental implant. The artificial joint prostesis may be ceramic femoral head for artificial hip joint, inlays for artificial hip joint, resurfacing femoral component, resurfacing acetabular component, or femoral component for artificial knee joint. The dental implant may be fixtures, superstructures, or abutments.

The present invention further includes ceramic parts that passed inspection by the strength proof test method.

The present invention further includes a device to be used in the strength proof test method for femoral head for artificial hip joint, comprising: two or more acoustic emission sensors to be attached in place of the femoral head ; a rubber member; and a tapered split segment capable of being split along the axial direction thereof, wherein the tapered split segment is disposed in contact, through the intermediary of the rubber member, with the inside of a cavity of the femoral head in such a manner that the tapered portion is directed downwardly in the vertical direction, which femoral head is to be disposed with the cavity being directed upwardly in the vertical direction, and the tapered split segment is made of a resin or a metal, or a composite material made of a resin and a metal, and wherein the cavity of the femoral head is pushed out by the pressure of a liquid medium, or mechanically, through the intermediary of the tapered slit segment.

The device may preferably comprise four or more acoustic emission sensors, and the sensors may preferably at least comprise one to be attached to the lowest portion at the outside of the femoral head and three to be attached at equal intervals in the vicinity of the cavity at the outside of the femoral head.

### EFFECTS OF THE INVENTION

The strength proof test method of the present invention makes it possible to improve the reliability of a strength proof test because parts causing the occurrence of damage in the strength proof test can be removed by an acoustic emission measurement.

### BRIEF DESCRIPTION OF DRAWINGS

Fig. 1(a) is an overall schematic view showing one example of the device of the present invention. Fig. 1(b) is an enlarged view of the split segment portion. Fig. 1(c) is an axial direction cross-sectional view of the split segment and the femoral head.
Fig. 2(a) is a schematic view of the four-point bending test and AE measurement device. Fig. 2(b) is a graph showing one example of the AE measurement results.
Fig. 3 is a bar graph showing, by type, the average values of residual strength measured after the strength proof test was carried out.
Fig. 4 is a Weibull plot of residual strength measured after the strength proof test was carried out.
Fig. 5 is a view showing a frame format of the outline of the AE method when the strength proof test of femoral heads is carried out.
Fig. 6 is a view showing the results of analyzing the stress distribution state by the finite element method for femoral heads for artificial hip joint.

### MODE FOR CARRYING OUT THE INVENTION

The present invention is characterized in that an acoustic emission (hereinafter called as "AE") measurement is made in the strength proof test for making total inspection of ceramic parts before use and the parts shall be considered as passing when the variation of AE energy is not more than a threshold.

The previous proof tests examine the presence or absence of defect in the proof tests and consider the possibly undamaged products as passing; however, it cannot be denied that the products, even if considered as passing, may possibly be fractured in actual use. The present invention examines the presence or absence of defect and further makes an AE measurement in the proof test to determine the products as passing when the variation of detected AE energy is not more than a threshold. Therefore, the present invention can remove products causing defect occurrence not less than a prescribed level in the proof test and can increase reliability more than the previous tests. Furthermore, the use of two or more AE sensors makes it possible to identify the location of defect occurrence from a difference of time for AE signals to reach the respective sensors, and therefore, friction between specimens in the measurement and noise occurring from the device can be removed to make a precious measurement. The number of AE sensors to be used may more preferably be not smaller than 3, still more preferably not smaller than 4, and usually not greater than 8.

The method of determining a load in the strength proof test may include the one described below, in which a test similar to the strength proof test of the present invention is carried out as a preliminary test by application of various loads to a prescribed number of ceramic components, and the results of the preliminary test can be used as the basis of determining a load in the strength proof test.

In the method based on the results of the preliminary test described above, a load to be applied in the proof test may be determined in view of a load resistant strength to be assured. For example, the stress by which main crack occurs in not less than 50% (preferably not less than 70% and particularly not less than 80%) of products subjected to the preliminary test (i.e., the stress at the AE rapidly increasing point) may be determined as a load to be applied in the strength proof test. Such load determination makes it possible to assure high reliability.

Furthermore, the finite element method may preferably be used in the strength proof test method of the present invention. In the method using the finite element method in combination, the ceramic parts are analyzed for the stress distribution state in actual use by the finite element method, and the stress distribution state can be used as the basis of determining a stress concentration region in actual use.

When the finite element method is used in combination, the products may be considered as passing when the AE energy detected from the stress concentration region analyzed from the stress distribution state is not more than a threshold. In the AE measurement, friction between the specimens or noise occurring from the device may sometimes prevent the accurate measurement. As described above, the determination of passing by the AE energy detected from the stress concentration region analyzed by the finite element method makes it possible to remove AE signals occurring in regions having no direct influence to the load resistant strength of members in actual use, i.e., regions other than the stress concentration regions found by the stress distribution state analysis, from AE signals to be evaluated, thereby further improving the reliability of measurement.

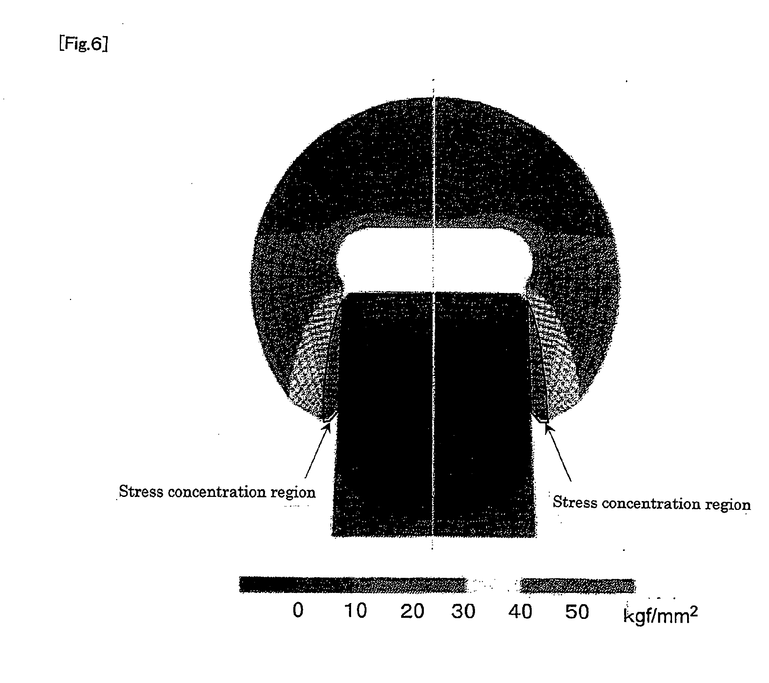

Fig. 6 shows one example of the analysis results of stress distribution state by the finite element method. The results shown in Fig. 6 were obtained by analyzing femoral heads for artificial hip joint for stress distribution state in actual use by the finite element analysis. In Fig. 6, the portions indicted by arrows are stress concentration regions, and the removal of AE signals occurring in regions other than the stress concentration regions, from those for evaluation, makes it possible to more improve the accuracy of measurement.

The material of ceramic parts to be subjected to the inspection in the present invention may be selected from almost all dense ceramics. Typical examples of such a material may include alumina, zirconia, silicon carbide, silicon nitride, apatite, soda glass, silica glass, bioglass, and composite ceramics thereof. The applications of ceramic parts to be subjected to the inspection in the present invention may be selected from all the products (parts) required to have high reliability on mechanical strength, such as in vivo implant members, exterior parts of aircrafts or the like, and various engine parts. Examples of the in vivo implant members may include artificial joint prosthesis and dental implant. The artificial joint prosthesis may are ceramic femoral heads for artificial hip joint, inlays for artificial hip joint, resurfacing femoral component, resurfacing acetabular component, or femoral component for artificial knee joint. The dental implant may specifically are fixtures, superstructures, or abutments.

The ceramic parts considered as passing by the method of the present invention can have about 5% to 20% higher strength as compared with the ceramic parts considered as passing by the proof tests that have hitherto been carried out. The present invention further includes ceramic parts considered as passing in such a manner by the strength proof test method of the present invention. The ceramic parts considered as passing by the strength proof test method of the present invention may have an average strength of not lower than 620 MPa, preferably not lower than 630 MPa, and more preferably not lower than 640 MPa, for example, in the case of high-purity alumna (having a purity of 99.9% or higher), and the upper limit of the strength is usually about 750 MPa.

The strength proof test method of the present invention can be carried out by the use of a device comprising a load applying system to apply a load to ceramic parts as the test piece, at least one AE sensor, and a signal processing system to detect and record a signal from the AE sensor. In particular, the present invention further includes a device to be used in the strength proof test method, particularly for femoral heads for artificial hip joint, among ceramic parts. The compression test of femoral heads for artificial hip joint can be carried out, usually by the method standardized in ISO7206. However, significant friction may occur between femoral heads and the load applying rod to produce much mechanical noise, and therefore, an AE measurement can hardly be carried out in the compression test.

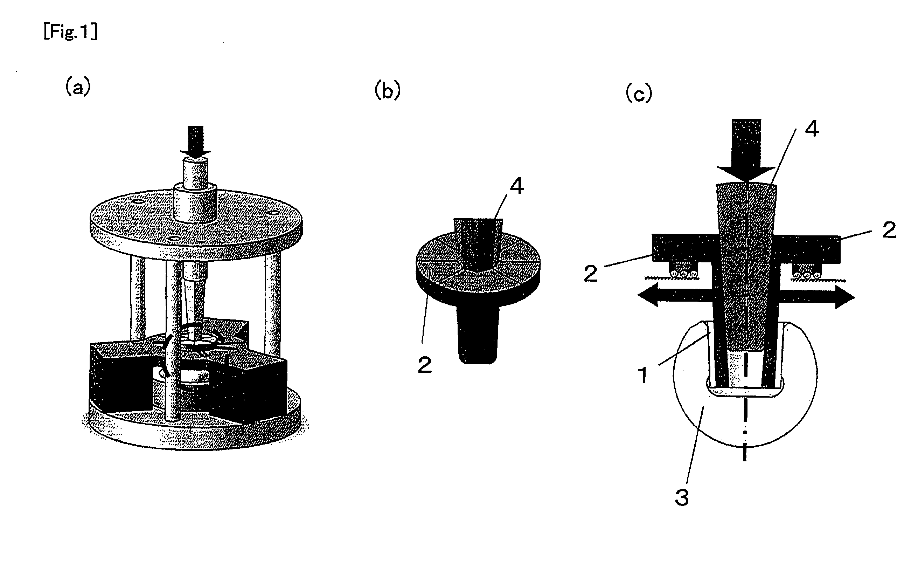

Fig. 1 shows one example of the strength proof testing device for femoral heads for artificial hip joint according to the present invention. In Fig. 1, the device comprises two or more acoustic emission sensors (not shown) to be attached in place of the femoral head, a rubber member 1, a tapered split segment 2 capable of being split along the axial direction thereof, and a tapered rod 4 having the same taper angle as that of the tapered split segment 2 and being to be inserted in the inside of the split segment 2 (Figs. 1(b) and 1(c)). In the present invention, the cavity of the femoral head can also be pushed out by the pressure of a liquid medium (e.g., water, compressor oil) instead of the tapered rod 4. When a liquid medium is used, a tool made of a polymer material such as polyethylene and a metal stopper, which has the same outer shape as that of the tapered rod and is hollowed out, may be used, for example, and pushed out from the inside of the polymer material by the pressure of the liquid medium, thereby generating force to push out the femoral head.

The tapered split segment 2 is disposed in contact, through the intermediary of the rubber member 1, with the inside of a cavity of the femoral head 3 in such a manner that the tapered portion is directed downwardly in the vertical direction, which femoral head is to be disposed with the cavity being directed upwardly in the vertical direction (Fig. 1(c)). The tapered split segment 2 is made of a resin or a metal, or a composite material of a resin and a metal, and the tapered split segment 2 can reduce friction that may possibly occur between the stress applying means (some mechanical method or some method by the pressure of a liquid medium) and the cavity of the femoral head, thereby making it possible to prevent noise generation in the AE measurement. The tapered split segment 2 may preferably have lower friction coefficient, and may preferably be made of a material such as Teflon (registered trademark) or polyethylene.

The split number of the tapered split segment 2 is not particularly limited, but may be about 2 to 8, more preferably 4 to 6. The tapered split segment 2 has a shape such as a hollow truncated cone, and the taper angle (i.e., angle between the center line and the edge line) can freely be determined, based on the taper angle of the test piece (product).

The tapered split segment 2 is disposed, through the intermediary of the rubber member 1, in the inside of a cavity of the femoral head 3, thereby making it possible to uniformly apply the stress, which was applied to the inside of the cavity, to femoral head 3. The rubber member 1 may be made of urethane rubber, silicone rubber, or ordinary vulcanized rubber or others.

The number of acoustic emission sensors is usually two or more, preferably four or more. When four or more sensors are used, the sensors may preferably at least comprise one to be attached to the lowest portion at the outside of the femoral head and three to be attached at equal intervals in the vicinity of the cavity at the outside of the femoral head. This makes it possible to more accurately determine the location of defect occurrence in the femoral head.

The present application claims the benefit of priority from Japanese Patent Application No. 2011-96038, filed on April 22, 2011. The entire contents of Japanese Patent Application No. 2011-96038, filed on April 22, 2011, are incorporated herein by reference.

### EXAMPLES

The present invention will hereinafter be described more specifically by way of Examples, but the present invention is not limited to the following Examples. The present invention can be put into practice after appropriate modifications or variations within a range meeting the gist described above and below, all of which are included in the technical scope of the present invention.

### Example 1

### (1) Consideration of applied load in the strength proof test

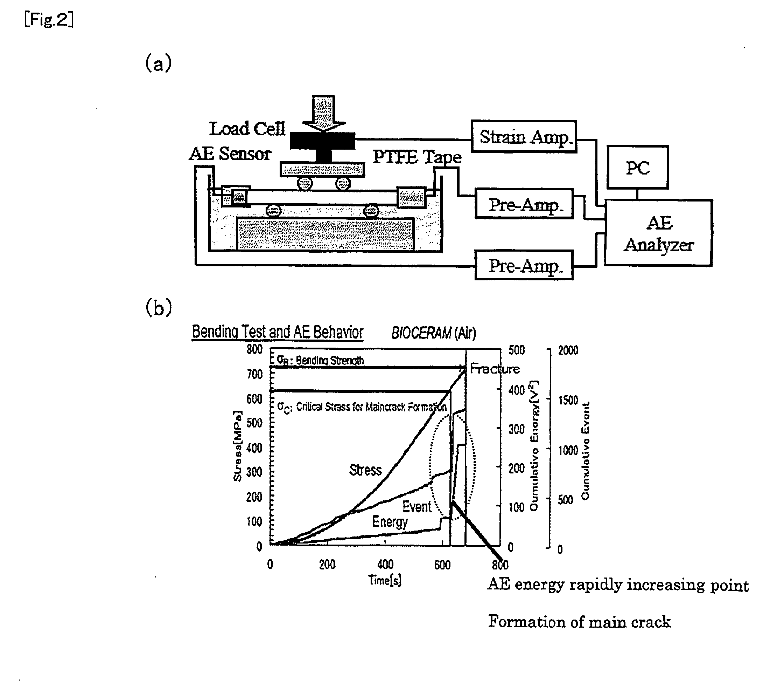

A bending test was carried out using high-purity alumina (having a purity of 99.9% or higher). Test pieces have a size of 3 mm x 4 mm x 40 mm, and the surface of each test piece was mirror-finished. As the bending test was adopted a four-point bending test (10 mm in inner span and 30 mm in outer span) in conformity to JIS R1601, as shown in Fig. 2(a), and the test was carried out in water. To both ends of each test piece were attached AE sensors, and the occurrence of defect in the test piece was monitored by the AE method. The AE measurement conditions were as follows: the gain of a preamplifier, 60 dB; the measurement threshold, 18 µV in terms of preamplifier input power; and the measurement frequency region, 95 to 660 kHz.

First, 129 test pieces were subjected to the four-point bending test at a crosshead speed of 0.1 mm/min to measure main crack forming critical stress σ_{c} (the stress at the point of rapidly increasing cumulative AE energy) and fracture strength σ_{B}. In addition, the location of finding AE energy was identified (location identification). The location identification can be achieved by a difference in the time of arrival of the detected AE wave at the respective sensors, together with the speed of sound in the test piece; however, some errors may be observed when the AE wave has small amplitude. For this reason, the AIC (Akaike Information Criteria) method was used to calculate a more accurate difference in the time of arrival, as described in "Naoki MAEDA: Reading and Evaluation in Earthquake Wave Automatic Processing System, Earthquake, Vo. 38 (1985), 365-379" and other literatures.

As a result, a cumulative AE energy rapidly increasing point was found before the final facture as shown in Fig. 2(b). Low energy signals were generated in the initial stage of the strength proof test, and large signals were then generated at the rapidly increasing point. In addition, after the observation of the rapidly increasing point, the accumulation of large AE signals was found in the vicinity of the final facture location of the test piece.

The average values of fracture strength σ_{B} and main crack forming critical stress σ_{C} were 579 MPa and 138 MPa, respectively. From the test results, the load to be applied in the strength proof test was determined to be 100 MPa and 200 MPa. The value of 100 MPa is sufficiently lower than the average value of fracture strength σ_{B} and corresponds to the stress by which main cracks were generated in 50% of all the test pieces, while the value of 200 MPa corresponds to the stress by which main cracks were generated in 80% of all the test pieces.

### (2) Strength proof test

The strength proof test was carried out by making a four-point bending test and AE measurement in the same manner as described in (1) above; however, a load was applied up to 100 MPa or 200 MPa at a crosshead speed of 0.1 mm/min, kept for 10 sec, 1 min, 3 min, or 10 min at 100 MPa or 200 MPa, and then removed at a crosshead speed of 0.2 mm/min. Subsequently, the respective test pieces after completion of the four-point bending test (the strength proof test) were subjected to another four-point bending test and AE measurement at a crosshead speed of 0.1 mm/min to measure fracture strength (residual strength).

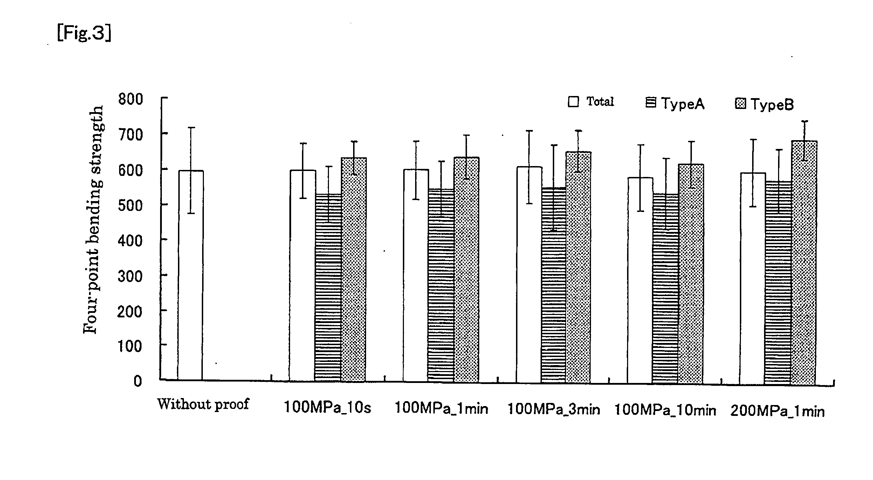

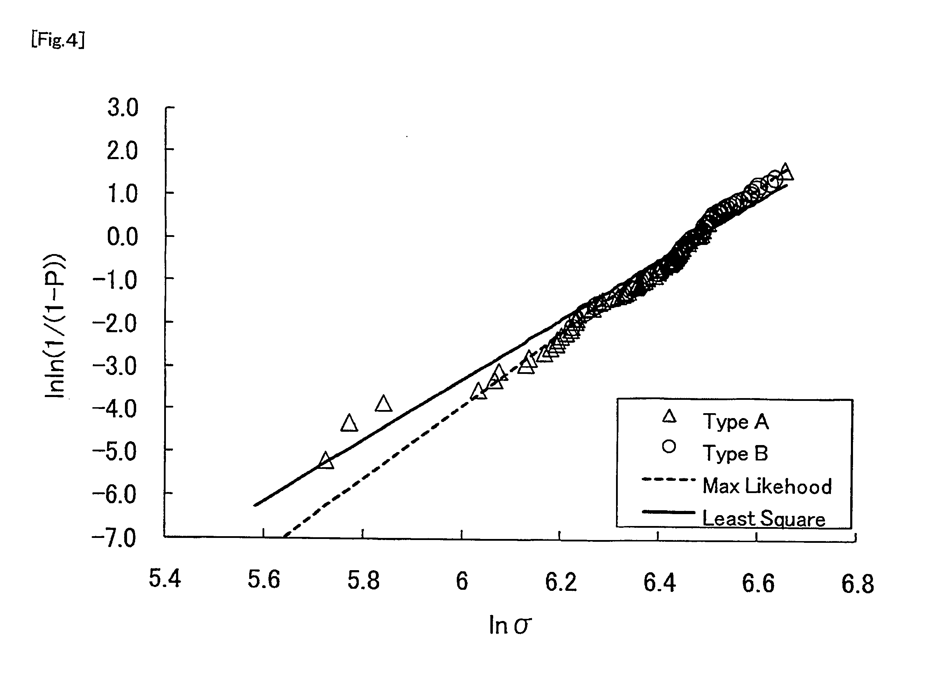

The results are shown in Tables 1, 2 and Figs. 3, 4. In these tables and figures, Type A means the test piece for which the AE energy rapidly increasing point was found in the strength proof test, and Type B means the test piece for which the AE energy rapidly increasing point was not found in the strength proof test. Table 1 shows the load applying conditions and the details of the test results, and Table 2 shows the average strength by type in each test condition. Fig. 3 shows the data of Table 2 by bar graph. Fig. 3 also shows the results when 15 test pieces were measured only for fracture strength in the four-point bending test without being subjected to the strength proof test. Fig. 4 is a Weibull plot of residual strength for the Type A and Type B pieces in all the test conditions.

**[Table 1]**

| Load applying conditions | | 100 MPa 10s | 100 MPa 1 min | 100 MPa 3 min | 100 MPa 10 min | 200 MPa 1 min |
|---|---|---|---|---|---|---|
| Number of test pieces | | 25 | 28 | 25 | 26 | 25 |
| Number of fracture in test | | 0 | 0 | 0 | 0 | 0 |
| Type A | Total number | 9 | 12 | 11 | 12 | 20 |
| | During load application | 6 | 7 | 9 | 9 | 17 |
| | During keeping | 0 | 3 | 2 | 3 | 1 |
| | During load removal | 3 | 2 | 0 | 0 | 2 |
| Type B | | 16 | 16 | 14 | 14 | 5 |

**[Table 2]**

| | 100 MPa 10 s | 100 MPa 1 min | 100 MPa 3 min | 100 MPa 10 min | 200 MPa 1 min |
|---|---|---|---|---|---|
| Total number | 599.3 ± 77.7 MPa | 603.5 ± 82.5 MPa | 615.4 ± 103.1 MPa | 587.5 ± 93.4 MPa | 604.6 ± 96.3 MPa |
| Type A | 532.3 ± 78.2 MPa | 550.9 ± 79.1 MPa | 558.4 ± 121.2 MPa | 541.3 ± 101.7 MPa | 581.5 ± 91.0 MPa |
| Type B | 636.9 ± 46.9 MPa | 642.9 ± 61.5 MPa | 660.3 ± 58.5 MPa | 627.1 ± 66.0 MPa | 697.2 ± 54.3 MPa |

From these results, it was found that Type A has lower residual strength than that of Type B and the removal of Type A considered as rejected makes it possible to remove low strength members that were not able to be removed by the previous proof tests determining only by the presence or absence of fracture.

### Example 2

In this Example, the compression test of femoral heads for artificial hip joint was carried out using a device shown in Fig. 1. The device shown in Fig. 1 comprises acoustic emission sensors (not shown) to be attached in place of the femoral head, a rubber member 1, a tapered split segment 2 capable of being split along the axial direction thereof, and a tapered rod 4 having the same taper angle as that of the tapered split segment 2 and being to be inserted in the inside of the split segment 2. In this Example, the rubber member 1 is made of urethane rubber.

The tapered split segment 2 is disposed in contact, through the intermediary of the rubber member 1, with the inside of a cavity of the femoral head 3 in such a manner that the tapered portion is directed downwardly in the vertical direction, which femoral head is to be disposed with the cavity being directed upwardly in the vertical direction. As the femoral head was used an alumina femoral head (having a purity of 99.9% or higher) for actual clinical use. The tapered split segment 2 has a hollow truncated cone shape, the material of which are stainless steel as a core material and a Teflon sheet lining on the face to be brought in contact with the femoral head, and the split number of which is 6.

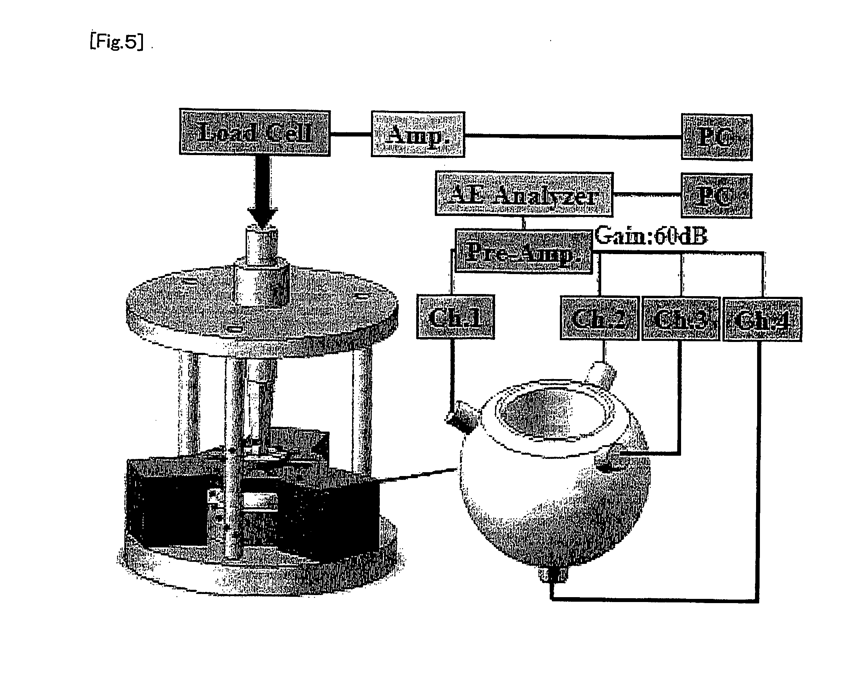

The tapered rod 4 is pushed in the axial direction thereof to open the tapered split segment 2 in the radius direction thereof, thereby applying stress to femoral head 3. The acoustic emission sensors were attached directly to the test piece as shown in Fig. 5, more specifically one AE sensor was attached to the lowest portion at the outside of the femoral head and three AE sensors were attached at equal intervals in the vicinity of the cavity at the outside of the femoral head, and monitoring was carried out by the AE method. As shown in Fig. 5, the AE signals obtained from the AE sensors were amplified through a preamplifier and then transferred to an AE analyzer. The AE measurement conditions were as follows: the measurement gain, 60 dB; the measurement threshold, 45 dB (about 0.18 V in terms of preamplifier input power); and the measurement frequency, 200 to 1200 kHz. Before the test, the tapered split segment 2, the tapered rod 4, and the rubber member 1 were subjected to ultrasonic cleaning and drying in air. The respective boundaries between the tapered split segment 2 and the tapered rod 4, between the split parts of the tapered split segment 2, and between the tapered split segment 2 and the rubber member 1, had been coated with Teflon for friction reduction.

As a result of the test with the device described above, a cumulative AE energy rapidly increasing point was found before the final fracture. A rapidly increase in cumulative AE energy was found at around about 700 seconds. As a result of identifying the location of obtaining a high amplitude AE signal at that time, it was found that the location of obtaining the AE signal occurrence substantially corresponds to the final fracture location of the femoral head, which makes it possible to find damage by the AE method even in the product shape of femoral heads.

### INDUSTRIAL APPLICABILITY

The strength proof test of the present invention is useful because it makes it possible to prevent the release of ceramic parts having damage introduced therein to the market, thereby improving the strength reliability of ceramic parts during use.

### EXPLANATION OF REFERENCE NUMERALS

- 1: Rubber member
- 2: Tapered split segment
- 3: Femoral head
- 4: Tapered rod

## Claims

1. A strength proof test method for making total inspection of ceramic parts before use, comprising:
applying a prescribed load to the ceramic parts; and
at the same time, making an acoustic emission measurement, in which the ceramic parts shall be considered as passing when the ceramic parts are not fractured in the test and the variation of acoustic emission energy is not more than a threshold.

2. The strength proof test method according to claim 1, wherein the location of acoustic emission occurrence is identified using two or more acoustic emission sensors.

3. The strength proof test method according to claim 1 or 2, wherein the ceramic parts are analyzed for stress distribution state in actual use by the finite element method to determine a stress concentration region in the ceramic parts, and
the ceramic parts shall be considered as passing when the ceramic parts are not fractured in the test and the acoustic emission energy occurring in the stress concentration region is not more than a threshold.

4. The strength proof test method according to any of claims 1 to 3, wherein the ceramic parts are in vivo implant members.

5. The strength proof test method according to claim 4, wherein the in vivo implant members are artificial joint components or dental implant members.

6. The strength proof test method according to claim 5, wherein the artificial joint components are ceramic femoral heads for artificial hip joint, inlays for artificial hip joint, resurfacing femoral component resurfacing acetabular component, or femoral component for artificial knee joint.

7. The strength proof test method according to claim 5, wherein the dental implant members are fixtures, superstructures, or abutments.

8. Ceramic parts that passed inspection by the strength proof test method according to any of claims 1 to 7.

9. A device to be used in the strength proof test method for femoral heads for artificial hip joint, comprising:
two or more acoustic emission sensors to be attached in place of the femoral head; a rubber member; and a tapered split segment capable of being split along the axial direction thereof,
wherein the tapered split segment is disposed in contact, through the intermediary of the rubber member, with the inside of a cavity of the femoral head in such a manner that the tapered portion is directed downwardly in the vertical direction, which femoral head is to be disposed with the cavity being directed upwardly in the vertical direction, and the tapered split segment is made of a resin or a metal, or a composite material made of a resin and a metal, and
wherein the cavity of the femoral head is pushed out by the pressure of a liquid medium, or mechanically, through the intermediary of the tapered slit segment.

10. The device according to claim 9, wherein the device comprises four or more acoustic emission sensors, and
the sensors at least comprise one to be attached to the lowest portion at the outside of the femoral head and three to be attached at equal intervals in the vicinity of the cavity at the outside of the femoral head.
